(19) Europäisches Patentamt
European Patent Office
Office européen des brevets

(11) Veröffentlichungsnummer: **0 244 723**
**A2**

# (12) EUROPÄISCHE PATENTANMELDUNG

(21) Anmeldenummer: **87106067.9**

(22) Anmeldetag: **25.04.87**

(51) Int. Cl.4: **C 07 D 279/16**, C 07 D 417/12, C 07 D 417/04, C 07 D 417/06, C 07 D 417/14, A 61 K 31/54 // C07D295/18, C07D295/22, C07C103/365

(30) Priorität: **28.04.86 DE 3614363**

(43) Veröffentlichungstag der Anmeldung: **11.11.87 Patentblatt 87/46**

(84) Benannte Vertragsstaaten: **AT BE CH DE ES FR GB GR IT LI LU NL SE**

(71) Anmelder: **HOECHST AKTIENGESELLSCHAFT, Postfach 80 03 20, D-6230 Frankfurt am Main 80 (DE)**

(72) Erfinder: **Lerch, Ulrich, Dr., Vorderwart 24, D-6238 Hofheim am Taunus (DE)**
Erfinder: **Henning, Rainer, Dr., Rotenhofstrasse 31, D-6234 Hattersheim am Main (DE)**
Erfinder: **Kaiser, Joachim, Dr., Fichtestrasse 12, D-6000 Frankfurt am Main (DE)**

## (54) Benzothiazinon-Derivate, Verfahren zu ihrer Herstellung, sie enthaltende Arzneimittel und deren Verwendung.

(57) Verbindung der Formel I

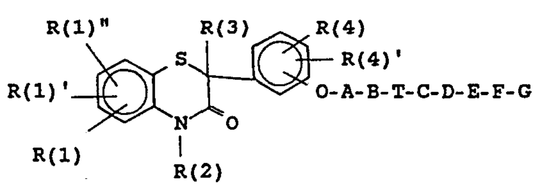

I

sowie Verfahren zu deren Herstellung, Arzneimittel mit calcium-antagonistischen Eigenschaften und die Verwendung dieser Verbindungen I als Arzneimittel werden beschrieben.

Die Substituenten haben darin folgende Bedeutung:
R(1) und R(4) H, Alkyl, Alkoxy, Halogen, $CF_3$, $NO_2$, OH, Acetamido oder Amino,
R(2) H, Alk(en)yl, Phenylalkyl;
R(3) H, (Cyclo-)Alk(en)yl, Cycloalkyl-alkyl, Phenyl, Phenylalkyl;
A $(CH_2)_{1-4}X-(CH_2)_{0-3}$ mit X gleich $CH_2$, O, S, CO, CHOH oder CR(5)R(5)'; und R(5) H oder Alkyl;
B eine Reihe von (Di)Aminen,
T $(CH_2)_{0-3}$;
D CHOH, CO, NR(10)CO, CONR(11), O;
E $(CH_2)_{0-3}$;
F eine Bindung, NR(12)CO, CONR(13), CO, O;
G Phenyl oder Furyl;
R(6), R(7), R(8), R(9) H, Alkyl, (Phenyl-)Alkanoyl, Phenylalkyl, Benzhydryl(alkyl), Benzyl;
R(10), R(11), R(12) H, Alkyl.

EP 0 244 723 A2

Benzothiazinon-Derivate, Verfahren zu ihrer Herstellung, sie enthaltende Arzneimittel und deren Verwendung

Es ist bekannt, daß Verbindungen, die das Einströmen von Calcium-Ionen in Zellen behindern, als Therapeutika zur Behandlung von verschiedenen Krankheiten, insbesondere das Herz-Kreislauf-Systems beim Menschen und anderen Warmblütern eingesetzt werden können.

Benzothiazinon-Derivate mit calciumantagonistischer Wirkung sind in US 4 584 300 beschrieben; die dort aufgeführten Verbindungen sind in der 2-Position des Heterocyclus unsubstituiert.

Weiterhin finden sich Benzothiazinon-Derivate mit calciumantagonistischer Wirkung in der US 4 595 685. Dort werden Verbindungen beschrieben, die am 2-Phenylrest eine basische Ethergruppierung tragen, wobei der basische Stickstoff über eine geradkettige oder verzweigte Alkylkette mit dem Ethersauerstoff verbunden ist.

Es wurde nun überraschend gefunden, daß Verbindungen mit veränderter Seitenkette am 2-Phenylrest überlegene calciumantagonistische Eigenschaften aufweisen.

Die Erfindung ist daher auf Benzothiazinon-Derivate der Formel I gerichtet, die calciumantagonistische Wirkung aufweisen:

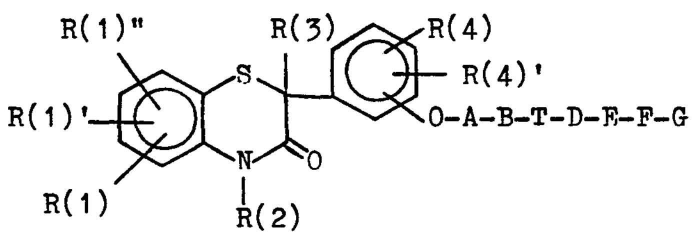

I

sowie deren Salze mit pharmazeutisch akzeptablen Säuren, in welcher Formel I bedeuten:

 

R(1), R(1)' und R(1)" gleich oder verschieden und voneinander unabhängig Wasserstoff, $(C_1-C_4)$-Alkyl, $(C_1-C_3)$-Alkoxy, F, Cl, Br, $CF_3$, Nitro, Hydroxy, Acetamido oder Amino,

R(2) Wasserstoff, $(C_1-C_{10})$-Alkyl, geradkettig oder verzweigt, $(C_3-C_{10})$-Alkenyl, geradkettig oder verzweigt, Phenyl-$(C_1-C_4)$-alkyl, wobei der Phenylring unsubstituiert oder durch einen, zwei oder drei Substituenten aus der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(3) Wasserstoff, $(C_1-C_{15})$-Alkyl, geradkettig oder verzweigt, $(C_3-C_{15})$-Alkenyl, geradkettig oder verzweigt, $(C_4-C_8)$-Cycloalkyl, $(C_4-C_8)$-Cycloalkyl-$(C_1-C_4)$alkyl, Phenyl oder Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist

R(4) und R(4)' gleich oder verschieden und voneinander unabhängig Wasserstoff, $(C_1-C_4)$-Alkyl, $(C_1-C_3)$-Alkoxy, F, Cl, $CF_3$, Nitro, Hydroxy, Acetamido oder Amino,

A die Gruppe $(CH_2)_m-X-(CH_2)_n$, wobei

m 1, 2, 3 oder 4, wobei jedoch m, wenn X ein Heteroatom ist, nur 2, 3 oder 4 ist,

n Null, 1, 2 oder 3, sofern X ein Heteroatom ist, jedoch nur 2 oder 3,

X eine $CH_2$-Gruppe, Sauerstoff, Schwefel, eine Carbonylgruppe, eine CH (OH)-Gruppe oder eine Gruppe

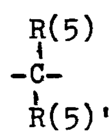 ,

worin

R(5) und R(5)' gleich oder verschieden sind und Wasserstoff oder $(C_1-C_4)$-Alkyl bedeuten,

B eine der folgenden Gruppen

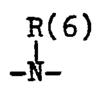 , 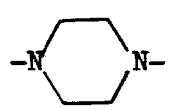 , 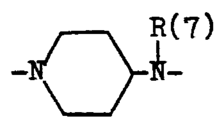

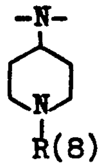 , 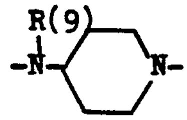 , 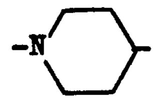

T einen $(CH_2)_p$-Rest, wobei p Null, 1, 2 oder 3 ist, wobei jedoch p nur 1, 2 oder 3 ist, wenn D die CHOH-Gruppe bedeutet, und wobei p nur 2 oder 3 bedeutet, wenn D ein Heteroatom bedeutet,

D eine CH(OH)-Gruppe, eine Carbonylgruppe, eine -NR(10)-CO-Gruppe, eine CO-NR(11)-Gruppe oder ein Sauerstoffatom,

E einen $(CH_2)_q$-Rest, wobei q Null, 1, 2 oder 3 ist, oder einen -CH=CH-Rest, jedoch, wenn D und/oder F ein Heteroatom bedeutet, E nur $(CH_2)_q$ mit q= 2 oder 3 bedeutet,

F eine Einfachbindung, einen NR(12)-CO-Rest, einen CO-NR(13)-Rest, eine Carbonylgruppe oder ein Sauerstoffatom,

G Phenyl, wobei der Phenylring unsubstituiert oder durch einen, zwei oder drei Substituenten der Gruppe $(C_1-C_4)$-Alkyl, $(C_1-C_3)$-Alkoxy, F, Cl, $(C_1-C_2)$-Alkylendioxy oder Cyano substituiert ist, oder 2-Furyl,

R(6) und R(7) und R(8) und R(9) Wasserstoff, $(C_1-C_{10})$-Alkyl, geradkettig oder verzweigt, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl, oder Benzyl, wobei die Phenylreste jeweils unsubstituiert oder durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(10) und R(11) und R(12) und R(13) Wasserstoff oder $(C_1-C_5)$-Alkyl;

Bevorzugt werden die Verbindungen der Formel I, in welcher mindestens einer der Substituenten und Indices folgende Bedeutung hat:

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido,

R(1)" Wasserstoff,

R(2) Wasserstoff, $(C_1-C_6)$-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, $(C_1-C_{12})$-Alkyl, geradkettig oder verzweigt, Allyl, Methallyl, $(C_5-C_7)$Cycloalkyl, $(C_5-C_7)$-Cycloalkyl-$(C_1-C_4)$-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)' Wasserstoff,

A die Gruppe $(CH_2)_m-X-(CH_2)_n$, wobei

m 1, 2, 3 oder 4, wobei jedoch m, wenn X ein Heteroatom ist, nur 2, 3 oder 4 ist, Heteroatom ist,

n Null, 1, 2 oder 3, sofern X ein Heteroatom bedeutet, jedoch nur 2 oder 3,

X eine $CH_2$-Gruppe, Sauerstoff, Schwefel, eine Carbonylgruppe eine CH(OH)-Gruppe oder eine Gruppe R(5)-C-R(5'), worin R(5) und

R(5)' gleich oder verschieden sind und Wasserstoff, Methyl oder Ethyl bedeuten

B eine der folgenden Gruppen

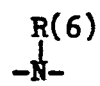 , 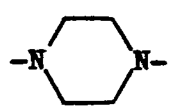 , 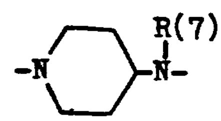

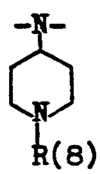 , 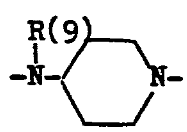 , 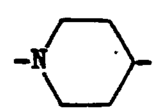

T einen $(CH_2)_p$-Rest, wobei p Null, 1 oder 2 ist, wobei jedoch p, wenn D die CHOH-Gruppe bedeutet, nur 1, 2 oder 3 ist, und wobei p, wenn D ein Heteroatom bedeutet, nur 2 oder 3 bedeutet,

D eine CH(OH)-Gruppe, eine Carbonylgruppe, eine -NR(10)-CO-Gruppe, eine CO-NR(11)-Gruppe oder ein Sauerstoffatom,

E einen $(CH_2)_q$-Rest, wobei q Null, 1 oder 2 ist, oder einen -CH=CH-Rest,

F eine Einfachbindung, einen NR(12)-CO-Rest, einen CO-NR(13)-Rest, eine Carbonylgruppe oder ein Sauerstoffatom,

G Phenyl oder 2-Furyl, wobei der Phenylring unsubstituiert oder durch ein oder zwei Substituenten aus der Gruppe $(C_1-C_2)$-Alkyl, F, Cl, $(C_1-C_2)$-Alkylendioxy oder Cyano oder durch ein, zwei oder drei $(C_1-C_2)$-Alkoxy-Substituenten substituiert ist,

R(6), R(7), R(8), R(9), R(10), R(11) und R(12) und R(13) Wasserstoff oder $(C_1-C_4)$-Alkyl,

sowie die Salze dieser Verbindungen I mit pharmazeutisch akzeptablen Säuren.

Besonders bevorzugt werden Verbindungen der Formel I, in welcher mindestens einer der Substituenten und Indices folgende Bedeutung hat:

R(1) Wasserstoff, Methyl, Methoxy, Fluor oder Chlor,

R(1)' Wasserstoff oder Methoxy,

R(1)" Wasserstoff,

R(2) Wasserstoff, Methyl, Ethyl, Propyl, Isopropyl, Butyl, sec. Butyl, Isobutyl, Benzyl, Phenethyl,

R(3) Wasserstoff $(C_1-C_{12})$-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl

R(4) Wasserstoff, Methoxy, Methyl, Chlor, Nitro oder Hydroxy

R(4)' Wasserstoff

A die Gruppe $(CH_2)_m-X-(CH_2)_n$, worin

m 1, 2 oder 3 wobei jedoch m,wenn X ein Heteroatom ist, nur 2, 3 oder 4 ist,

n 0, 1 oder 2, sofern X ein Heteroatom bedeutet, jedoch nur 2

X eine $CH_2$-Gruppe, Sauerstoff, eine Carbonylgruppe eine CH(OH)-Gruppe oder eine Gruppe

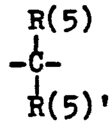

worin

R(5) und R(5)' gleich oder verschieden sind und Wasserstoff oder Methyl bedeuten,

B eine der folgenden Gruppen

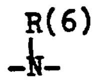 , 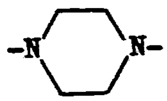 , 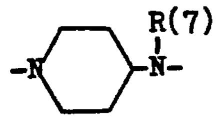

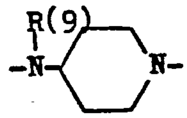 , 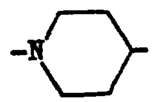

T einen $(CH_2)_p$-Rest, wobei p Null, 1 oder 2 ist, wobei jedoch p, wenn D die CHOH-Gruppe bedeutet, nur 1, 2 oder 3 ist, und wobei p, wenn D ein Heteroatom bedeutet, nur 2 oder 3 bedeutet,

D eine CH(OH)-Gruppe, eine Carbonylgruppe, eine -NR(10)-CO-Gruppe, eine CO-NR(11)-Gruppe oder ein Sauerstoffatom,

E ein $(CH_2)_q$-Rest, wobei q Null, 1 oder 2 ist,

F eine Einfachbindung, einen NR(12)-CO-Rest, einen CO-NR(13)-Rest, eine Carbonylgruppe oder ein Sauerstoffatom

G Phenyl, wobei der Phenylring unsubstituiert oder durch 1 oder 2 Reste der Gruppe Methyl, F, Cl, Methylendioxy oder Cyano oder durch 1, 2 oder 3 Methoxyreste substituiert ist, oder 2-Furyl

R(6), R(7), R(8), R(9), R(10), R(11), R(12) und R(13) gleich oder verschieden sind und Wasserstoff oder $(C_1-C_3)$-Alkyl bedeuten

sowie die Salze dieser Verbindungen I mit pharmazeutisch verträglichen Säuren.

Als solche pharmazeutisch akzeptablen Säuren kommen anorganische Säuren wie Salzsäure, Bromwasserstoffsäure, Jodwasserstoffsäure, Schwefelsäure, Phosphorsäure oder Salpetersäure oder organische Säuren wie Weinsäure, Äpfelsäure, Milchsäure,

Maleinsäure, Fumarsäure, Malonsäure, Oxalsäure, Gluconsäure, Camphersulfonsäure, Benzolsulfonsäure, Essigsäure, Propionsäure oder p-Toluolsulfonsäure in Betracht.

Die Verbindungen der Formel I weisen asymmetrische C-Atome auf und können daher als Enantiomere oder Diastereomere auftreten. Die Erfindung umfaßt sowohl die reinen Isomeren als auch deren Gemische. Diese Gemische von Diastereomeren können nach gebräuchlichen Methoden, zum Beispiel selektive Kristallisation aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung.

Die Erfindung betrifft weiterhin Verfahren zur Herstellung von Verbindungen der Formel I, dadurch gekennzeichnet, daß man

a) eine Verbindung der Formel II,

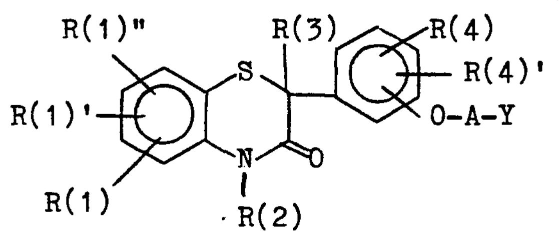

II,

in welcher R(1), R(1)', R(1)", R(2), R(3), R(4), R(4)' und A die gleiche Bedeutung wie in Formel I haben, und in welcher Y eine Abgangsgruppe, die nucleophil verdrängt werden kann, insbesondere ein Chlor-, Brom- oder Jodatom, einen Sulfonsäurerest, vorzugsweise einen Methansulfonylrest, einen Benzolsulfonylrest, einen Toluolsulfonylrest oder einen Trifluormethansulfonylrest bedeutet, mit einer Verbindung der Formel III

H-B-T-D-E-F-G      III,

in welcher B, T, D, E, F und G die gleiche Bedeutung wie in Formel I haben, unter Bedingungen einer nucleophilen Substitution, vorzugsweise in einem polaren organischen Lösungsmittel wie einem Alkohol, vorzugsweise Methanol, Ethanol, Propanol oder Isopropanol oder einem niederen Keton, vorzugsweise Aceton oder Methylethylketon oder Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumcarbonat, Natriumcarbonat, Triethylamin, N-Ethylmorpholin oder Pyridin, bei einer Temperatur zwischen 0 und 160°C, vorzugsweise zwischen 20 und 120°C, umsetzt, oder daß man

b) eine Verbindung der Formel IV,

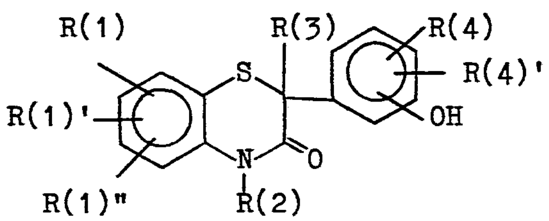

IV,

in welcher R(1), R(1)', R(1)", R(2), R(3), R(4) und R(4)' die gleiche Bedeutung wie in Formel I haben, mit einer Verbindung der Formel V,

Z-A-B-T-D-E-F-G V,

in welcher Z gleich wie Y in Formel II definiert ist und in welcher A, B, T, D, E, F und G die gleiche Bedeutung wie in Formel I haben, entweder in einem polaren aprotischen Lösungsmittel wie Dimethylformamid, Dimethylsulfoxid, Tetrahydrofuran, Sulfolan oder N-Methylpyrrolidon, in Gegenwart einer starken Base wie Natriumhydrid, Kaliumhydrid, Natriumamid, Lithiumdiisopropylamid, Butyllithium oder Lithiumhexamethyldisilazid, bei einer Temperatur

zwischen -40 und +60°C, vorzugsweise zwischen -10 und -30°C, oder in einem protischen oder aprotischen polaren organischen Lösungsmittel wie einem niederen Alkohol, beispielsweise Methanol, Ethanol, Isopropanol oder einem niederen Keton, vorzugsweise Aceton oder Methylethylketon oder in Dimethylformamid, in Gegenwart einer schwachen bis mittelstarken Base wie einem Alkali- oder Erdalkalimetallhydroxid oder -carbonat oder einem Amin wie beispielsweise Triethylamin, N-Ethylmorpholin, N-Methyldiisopropylamin oder Pyridin, bei einer Temperatur zwischen 0 und 160°C, vorzugsweise zwischen 20 und 120°C, umsetzt, oder daß man

c) eine Verbindung der Formel V,

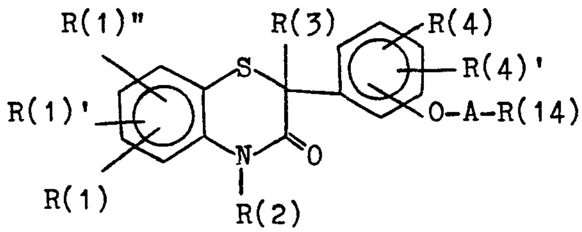

V

in welcher R(1), R(1)', R(1)", R(2), R(3), R(4), R(4)', und A die gleiche Bedeutung wie in Formel I haben, und in welcher R(14) eine der folgenden Gruppen

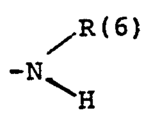 , 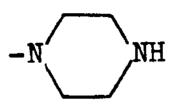 , 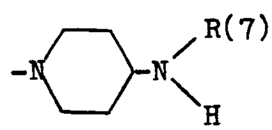 ,

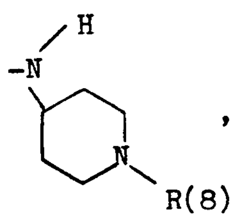 , 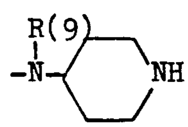

bedeutet, worin R(6), R(7), R(8) und R(9) die gleiche Bedeutung wie in Formel I haben, mit einer Verbindung der Formel VI

 

$$W-\underset{\overset{\|}{O}}{C}-E-F-G \qquad VI,$$

in welcher E, F und G die gleiche Bedeutung wie in Formel I haben, und W ein Chloratom, eine $(C_1-C_5)$-Alkoxygruppe, eine $(C_1-C_5)$-Alkanoyloxygruppe oder ein Imidazolrest ist, unter üblichen Amidbildungsbedingungen umsetzt, wobei Verbindungen der Formel I gebildet werden, in denen p Null und D eine Carbonylgruppe bedeuten, oder daß man

d) eine Verbindung der Formel VII

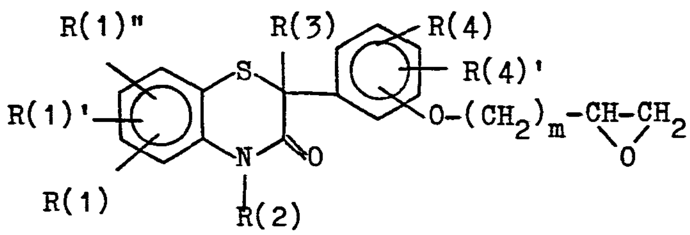

VII,

in welcher R(1), R(1)', R(1)", R(2), R(3), R(4), R(4)' und m die gleiche Bedeutung wie in Formel I haben, mit einer Verbindung der Formel VIII

H-B-T-D-E-F-G VIII,

in welcher B, T, D, E, F, G wie in Formel I definiert sind, ohne Lösungsmittel oder in Gegenwart eines vorzugsweise polaren Lösungsmittels wie Methanol, Isopropanol, Aceton, THF oder Dimethylformamid umsetzt, wobei Verbindungen der Formel I entstehen, worin A den Rest $-(CH_2)_m-\underset{OH}{CH}-CH_2-$ bedeutet, oder daß man

e) eine Verbindung der Formel V mit einer Verbindung der Formel IX

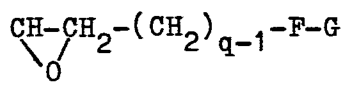

IX

worin F, G und q die gleiche Bedeutung wie in Formel I haben, unter den unter d) beschriebenen Reaktionsbedingungen umsetzt, wobei Verbindungen der Formel I gebildet werden, in denen p Null und D eine CH(OH)-Gruppe bedeuten.

Aus Verbindungen der Formel IV lassen sich durch Umsetzung mit Verbindungen der Formel X

$$Q-A-Y \qquad X$$

in welcher A die gleiche Bedeutung wie in Formel I, Y die gleiche Bedeutung wie in Formel II hat und Q gleich definiert ist wie Y in Formel II in Gegenwart von Basen wie z.B. Natriumhydroxyd, Kaliumcarbonat, Natriummethylat oder Kaliumtertiärbutylat in einem Lösungsmittel wie z. B. Tetrahydrofuran, Methanol, Dimethoxyethan, Aceton, Methylethylketon, Dimethylformamid oder Dimethylsulfoxid Verbindungen der Formel II herstellen.

Verbindungen der Formel VI erhält man aus Verbindungen der Formel IV z. B. mit Epichlorhydrin und Base (für m = 1) nach bekannten Methoden, oder durch Alkylierung von Verbindungen der Formel IV mit Verbindungen der Formel XI

$$Y-(CH_2)_m-CH=CH_2 \qquad XI$$

worin m die gleiche Bedeutung hat wie in Formel I und Y gleich definiert ist wie in Formel II, wobei Verbindungen der Formel XIII

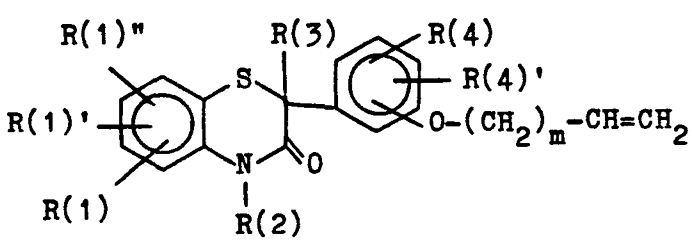

XIII

gebildet werden, worin R(1), R(1)', R(1)", R(2), R(3), R(4), R(4)' und m die gleiche Bedeutung wie in Formel I

besitzen. Anschließende Epoxidation der Verbindungen XIII nach bekannten Methoden, z.B. mit m-Chlorperbenzoesäure in Methylenchlorid ergibt Verbindungen der Formel VII.

Verbindungen der Formel V erhält man aus Verbindungen der Formel II durch Umsetzungen mit einer der Verbindungen der Formel XIIa, XIIb, XIIc, XIId oder XIIe

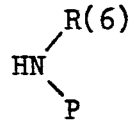 , 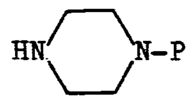 , 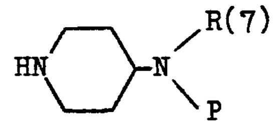 ,

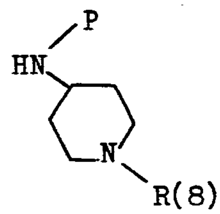 , 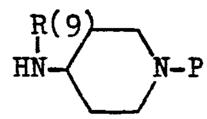 XIIa-e

in welchen R(6), R(7), R(8), R(9) die gleiche Bedeutung in Formel I haben, und in welcher P ein Wasserstoffatom, eine ($C_1$-$C_4$)-Alkoxycarbonylgruppe, eine Benzyloxycarbonylgruppe, eine Trifluoracetylgruppe oder eine Trichlormethylcarbonyloxygruppe bedeutet, unter den unter Verfahrensvariante a) beschriebenen Bedingungen und nachfolgende Abspaltung der Gruppe, falls diese nicht Wasserstoff bedeutet, nach literaturbekannten Verfahren.

Verbindungen der Formel IV sind aus EP-A-146 893 bekannt.

Die erfindungsgemäßen Verbindungen der Formel I weisen blutdrucksenkende, insbesondere calciumantagonistische Wirkungen auf und können daher zur Behandlung aller Krankheitszustände, die auf einer Störung in dem Calciumhaushalt eines Warmblüters beruhen, verwendet werden.

Ihre calciumantagonistische Wirksamkeit kann an dem biochemischen Testmodell der Verdrängung von tritiummarkiertem Nitrendipin gezeigt werden.

Hierbei werden Membranpräparationen, die isolierte Calciumkanäle enthalten, mit der markierten Substanz beladen. Nach Inkubation mit der Testsubstanz wird die freigesetzte Radioaktivität in der überstehenden Lösung bestimmt. In diesem Modell weisen die erfindungsgemäßen Verbindungen der Formel I $IC_{50}$-Werte von $10^{-6}$ molar bis $10^{-10}$molar auf. In weiteren Testmodellen, mit denen calciumantagonistische Wirkung nachgewiesen werden kann, z. B. an der Coronardurchströmung am isolierten Meerschweinchenherzen oder am Aktionspotential des isolierten Meerschweinchenpapillarmuskels sind die Verbindungen der Formel I ebenfalls stark wirksam.

Die erfindungsgemäßen Verbindungen der Formel I und ihre pharmakologisch verträglichen Salze vermindern das Einströmen von Calcium-Ionen in Zellen und eignen sich daher zu Behandlung des Herz-Kreislaufsystems bei entsprechenden Beschwerden z. B. bei verschiedenen Formen der Angina pectoris, Tachycardie, Herzrhythmusstörungen und Bluthochdruck. Sie sind innerhalb eines breiten Dosisbereichs wirksam. Die Höhe der verabreichten Dosis ist abhängig von der Art der gewünschten Behandlung, von der Verabreichungsweise, vom Zustand, vom Typ und von der Größe des behandelten Säugers. Bei oraler Dosierung werden befriedigende Ergebnisse mit Dosen von ab 0,01 mg, vorzugsweise ab 0,1 mg und bis zu 100 mg, vorzugsweise bis zu 20 mg einer Verbindung der Formel I pro kg Körpergewicht erreicht. Beim Menschen variiert die tägliche Dosis zwischen 10 und 800 mg, vorzugsweise 20 bis 500 mg, wobei Einzeldosen von 5 bis 200 mg, vorzugsweise ein bis dreimal täglich, gegeben werden können.

Für intravenöse und intramuskuläre Anwendung beträgt die Dosis 1 bis 300 mg, vorzugsweise 5 bis 150 mg täglich.

Die pharmakologisch verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z. B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z. B. mittels konventioneller Misch-Granulier- und Dragierverfahren, hergestellt und enthalten 0,1 % bis etwa 75 %, bevorzugt etwa 1 % bis etwa 50 % des Wirkstoffs.

Alkyl, Alkenyl, Alkoxy, Alkylen bedeuten, wenn nicht ausdrücklich anders angegeben, stets eine gerade oder verzweigte Kette.

Die im Anschluß folgenden Beispiele sollen die Erfindung erläutern, ohne sie auf diese Beispiele zu begrenzen.

0244723

Beispiel 1

3,4-Dihydro-2-isopropyl-4-methyl-2-[2-[4-[4-(2-(3,4,5-trimethoxyphenyl)-acetyl]-piperazinyl]-butoxy]-phenyl]-2H-1,4-benzothiazin-3-on-Hydrochlorid

6.68 g (15 mmol) 3,4-Dihydro-2-isopropyl-4-methyl-2-[2-(4-brombutoxy)-phenyl]-2H-1,4-benzothiazin-3-on wurden in 30 ml DMF mit 2,07 g Kaliumcarbonat und 6,63g (22,5 mmol) 3,4,5-Trimethoxyphenyl-essigsäurepiperazid 5 Stunden am Rückfluß gerührt. Nach Abkühlen gab man 250 ml Wasser zu, dekantierte das Wasser vom öligen Rückstand und nahm diesen in Essigester auf. Nach Waschen mit Wasser und Trocknen wurde eingeengt. Chromatographie an 250 g Kieselgel mit Methylenchlorid/Methanol (9:1) als Laufmittel ergab 2,4 g der Titelverbindung (freie Base) als Öl.

$^1$H-NMR ($CDCl_3$) : $\delta$ = 7,5 - 6,5 (m,8H); 6,45 (s,2H); 4,2 - 3,9 (m,2H); 3,8 (s,9H); 3,6 (s,2H); 3,4 (s,3H); 3,1 - 2,1 (m,11H); 2,0 - 1,5 (m,4H); 1,1 + 0,95 (2d,6H) ppm.

Das Hydrochlorid erhielt man durch Versetzen mit ethanolischer Salzsäure, Einengen und Verreiben mit Petrolether, 1,7 g, Schmp. 125°C

| | | | | | |
|---|---|---|---|---|---|
| Ber. | $C_{37}H_{45}N_3O_6S{\cdot}HCl$ | C 63,5 | H 6,9 | N 6,0 | Cl 5,1 |
| Gef. | | C 62,6 | H 7,1 | N 5,8 | Cl 5,5 |

Die Herstellung von 3,4-Dihydro-2-isopropyl-4-methyl-2-[2-(4-brombutoxy]-phenyl]-2H-1,4-benzothiazin-3-on ist in EP-A-146 893 beschrieben.

3,4,5-Trimethoxyphenylessigsäure-piperazid erhält man folgendermaßen:

11,3 g 3,4,5-Trimethoxyphenylessigsäure und 23,3 ml einer 30 %igen Lösung von 1-Hydroxybenztriazol wurden bei Zimmertemperatur mit 12,3 g Dicyclohexylcarbodiimid und 8,8 g N-Benzylpiperazin versetzt. Nach 15 Std. wurde abgesaugt und das Filtrat mit Wasser verdünnt. Nach dreimaligem Extrahieren mit Essigester wurden die vereinigten organischen Phasen je 1 x mit ges. $NaHCO_3$-Lösung, 10 %iger Citronensäure-Lösung und gesättigter Kochsalzlösung gewaschen, getrocknet und eingeengt. Das so erhaltene N-Benzyl-N'-Trimethoxyphenylacetyl)-piperazin wurde in 45 ml Methanol gelöst und mit 1 g Palladium auf Tierkohle (10 %) 8 Std. bei 80°C und 30 atm $H_2$-Druck hydriert. Nach Abfiltrieren wurde eingeengt und der Rückstand in Aceton gelöst und mit etherischer Salzsäure versetzt. Das Hydrochlorid wird abgesaugt. Ausbeute 5,7 g, Schmp. 108 - 110°C.

Beispiel 2

3,4-Dihydro-2-isopropyl-4-methyl-2-[2-[4-[4-(2-(3,4,5-trimethoxyphenyl)-propionyl]-piperazinyl]-butoxy]-phenyl]-2H-1,4-benzothiazin-3-on-Hydrochlorid

3,01 g (6,7 mmol) 3,4-Dihydro-4-methyl-2-[2-(4-brombutoxy)-phenyl]-2H,1,4-benzothiazin-3-on wurden mit 4,5 g 3-(3,4,5-Trimethoxyphenyl)-propionsäure-piperazid und 1,4 g Kaliumcarbonat in 100 ml Isopropanol 8 Std. zum Sieden erhitzt. Nach Abkühlen wurde filtriert, eingeengt und an Kieselgel mit Methylenchlorid/Methanol (9:1) chromatographiert. Man erhielt 3,9 g der freien Base als Öl.

$^1H$-NMR ($CDCl_3$) : $\delta$ = 7,6 - 6,5 (m,8H); 6,4 (s,2H); 3,85 + 3,82 (2s,9H); 4,2 - 3,3 (m,4H); 3,4 (s,3H); 3,0 - 2,0 (m,13H); 2,0 - 1,5 (m,4H); 1,15 - 0,9 (2d,6H) ppm.

3-(3,4,5-Trimethoxyphenyl)-propionsäure-piperazid erhielt man analog der für 3,4,5-Trimethoxyphenyl-essigsäure-piperazid angegebenen Vorschrift unter Verwendung von 3-(3,4,5-trimethoxyphenyl)-propionsäure als Ausgangsmaterial.

Beispiel 3

3,4-Dihydro-2-isopropyl-4-methyl-2-[2-(4-piperazinyl)-butoxyphenyl]-2H-1,4-benzothiazin-3-on-Dihydrochlorid

4,5 g (10 mmol) 3,4-Dihydro-isopropyl-4-methyl-2-[2-(4-brombutoxy)-phenyl]-2H,1,4-benzothiazin-3-on wurden in 50 ml Isopropanol mit 1,4 g Kaliumcarbonat und 2,58 g wasserfreiem Piperazin 16 Std. zum Sieden erhitzt. Nach Filtrieren wurden eingeengt, in einer Mischung aus Essigester und Toluol aufgenommen, dreimal mit Wasser gewaschen, mit Natriumsulfat getrocknet und eingeengt. Man erhielt 4,3 g der Titelverbindung als Öl (freie Base).

$^1$H-NMR ($CDCl_3$) : $\delta$ = 7,6 - 6,4 (m,8H); 3,9 (+,2H); 3,38 (s,3H); 3,0 + 2,4 (m,11H); 2,1 (s,1H); 1,9 - 1,5 (m,4H); 1,15 - 0,95 (2d,6H) ppm.

Dihydrochlorid: farblose Kristalle, Schmp. 110°C (Zers.)

Beispiel 4

3,4-Dihydro-2-isopropyl-4-methyl-2-[2-[4-[4-(2-furoyl)-piperazinyl]-butoxy]-phenyl]-2H-1,4-benzothiazin-3-on Hydrochlorid

4,5 g der Verbindung aus Beispiel 3 und 1 g Triethylamin wurden in 50 ml Methylenchlorid gelöst. Bei 0°C bis 5°C tropfte man 1,3 g Furan-2-carbonsäurechlorid zu. Nach 4 Std. wurde in Eiswasser gegossen, die org. Phase abgetrennt, mit gesättigter Natriumbicarbonat-Lösung und Wasser gewaschen und mit Natriumsulfat getrocknet. Man erhielt 4,3 g der freien Base als Öl.

$^1$H-NMR ($CDCl_3$) : $\delta$ = 7,6 - 6,3 (m,11H); 4,0 - 3,6 (m,6H); 3,36 (s,3H); 3,1 - 2,2 (m,7H); 2,0 - 1,6 (m,4H); 1,1 + 0,9 (2d,6H) ppm.

Das Hydrochlorid erhielt man aus methanolischer Lösung mit etherischer Salzsäure, Schmp. 186 - 187°C (Zers.)

| | | | | |
|---|---|---|---|---|
| Ber. $C_{31}H_{37}N_3O_4S\cdot HCl$ | C 63,75 | H 6,55 | N 7,2 | Cl 6,1 |
| Gef. | C 63,4 | H 6,6 | N 7,1 | Cl 6,2 |

## Beispiel 5

3,4-Dihydro-2-isopropyl-4-methyl-2-[2-[4-[4-(3,4,5-trimethoxycinnamoyl)-piperazinyl]-butoxy]-phenyl]-2H-1,4-benzothiazin-3-on-Hydrochlorid

4,6 g (10 mmol) der Verbindung aus Beispiel 3 wurden mit 5 ml einer 30 % Lösung von 1-Hydroxybenztriazol in DMF, 4,6 g Dicyclohexylcarbodiimid und 2,5 g 3,4,5-Trimethoxyzimtsäure 18 Stunden bei Zimmertemperatur gerührt. Nach Absaugen wurde auf Wasser gegossen, mit Essigester extrahiert, mit Wasser gewaschen, getrocknet und eingeeengt. Chromatographie an Kieselgel ergibt 3,8 g freie Base als Öl.

$^1$H-NMR ($CDCl_3$) : δ = 7,6 - 6,4 (m,12H); 4,6 - 3,6 (m,6H); 3,85 (s,9H); 3,4 (s,3H); 3,3 - 2,6 (m,7H); 2,0 - 1,6 (m,4H); 1,2 + 0,95 (2d,6H) ppm.

Hydrochlorid: Farblose Kristalle, Schmp. 193 - 194°C

| | | | | |
|---|---|---|---|---|
| Ber. $C_{38}H_{47}N_3O_6S\cdot HCl$ | C 64,3 | H 6,8 | N 5,9 | Cl 5,0 |
| Gef. | C 63,2 | H 6,8 | N 5,8 | Cl 5,0 |

## Beispiel 6

3,4-Dihydro-2-isopropyl-4-methyl-2-[2-[4-[4-(2-hydroxy-3-(2-cyano-phenoxy)-propyl]-piperazinyl]-butoxy]-phenyl]-2H-1,4-benzothiazin-3-on-Dihydrochlorid

4,6 g der Verbindung aus Beispiel 3 und 1,7 g 2-(2,3-Epoxypropoxy)-benzonitril wurden in 50 ml Methanol 3 Std. am

Rückfluß gerührt. Nach Einengen wurde an Kieselgel chromatographiert. Man erhielt 1,9 g freie Base als Öl.

$^1$H-NMR ($CDCl_3$) : δ = 7,6 - 6,5 (m,12H); 4,2 - 3,6 (m,5H); 3,4 (s,3H); 3,2 - 2,3 (m,13H); 2,0 - 1,6 (m,4H); 1,2 - 0,95 (2d,6H) ppm.

Dihydrochlorid: Farblose Kristalle, Schmp. 130 - 131°C (Zers.)

| | | | | | |
|---|---|---|---|---|---|
| Ber. | $C_{35}H_{44}N_4O_4S \cdot 2HCl$ | C 60,9 | H 6,7 | N 8,1 | Cl 10,3 |
| Gef. | | C 59,8 | H 6,7 | N 7,5 | Cl 10,1 |

Beispiel 7

3,4-Dihydro-2-isopropyl-4-methyl-2-[2-[4-[2-hydroxy-3-(2-chlor-phenoxy)-propyl]-piperazinyl]-butoxy]-phenyl]-2H-1,4-benzothiazin-3-on-Dihydrochlorid

4,6 g der Verbindung aus Beispiel 3 wurden mit 1,84 g 1-(2-Chlorphenoxy)-2,3-epoxypropan analog dem in Beispiel 6 beschriebenen Verfahren umgesetzt. Man erhielt 2,7 g der freien Base als Öl.

$^1$H-NMR ($CDCl_3$) : δ = 7,5 - 6,8 (m,12H); 4,2 - 3,6 (m,7H); 3,4 (s,3H); 3,1 - 2,3 (m,13H); 2,0 - 1,6 (m,4H); 1,15 + 0,95 (2d,6H) ppm.

Dihydrochlorid: Farblose Kristalle, Schmp. 195°C

| | | | | | |
|---|---|---|---|---|---|
| Ber. | $C_{35}H_{44}N_3O_4S \cdot 2HCl \cdot H_2O$ | C 57,7 | H 6,6 | N 5,8 | Cl 14,6 |
| Gef. | | C 57,7 | H 6,5 | N 5,5 | Cl 14,2 |

Beispiel 8

3,4-Dihydro-2-isopropyl-4-methyl-2-[2-[4-[4-[2-hydroxy-3-(2-methoxyphenoxy)-propyl]-piperazinyl]-butoxy]-phenyl]-2H-1,4-benzothiazin-3-on-Dihydrochlorid

4,6 g der Verbindung aus Beispiel 3 wurden mit 1,8 g 1-(2-Methoxy-phenoxy)-2,3-epoxy-propan analog dem in Beispiel 6 beschriebenen Verfahren umgesetzt. Man erhält 2,4 g der freien Base als Öl.

$^1$H-NMR ($CDCl_3$) : δ = 7,6 - 6,4 (m,12H); 4,2 - 3,6 (m,7H); 3,8 (s,3H); 3,4 (s,3H); 3,1 - 2,3 (m,13H); 2,0 - 1,6 (m,4H); 1,2 - 0,95 (2d,6H) ppm.

Dihydrochlorid: Farblose Kristalle, Schmp. 130 - 132°C (Zers.)

| | | | | | |
|---|---|---|---|---|---|
| Ber. | $C_{36}H_{47}N_3O_5S \cdot 2HCl \cdot 2H_2O$ | C 58,2 | H 7,2 | N 5,7 | Cl 9,6 |
| Gef. | | C 57,7 | H 6,8 | N 5,4 | Cl 9,1 |

## Beispiel 9

3,4-Dihydro-2-isopropyl-4-methyl-2-[2-[4-[4-(2,6-dimethylanilino-carbonyl)-methyl]-piperazinyl]-butoxy]-phenyl]-2H-1,4-benzothiazin-3-on-Dihydrochlorid

4,5 g (10 mmol) der Verbindung aus Beispiel 3 wurden mit 1,97 g (10 mmol) Chloressigsäure-2,6-dimethylanilid in 50 ml Methylenchlorid 10 Stunden bei Zimmertemperatur gerührt. Nach Einengen wird an 300 g Kieselgel mit Methylenchlorid/Methanol (9:1) chromatographiert. Man erhält 2,0 g der freien Base als Öl.

$^1$H-NMR ($CDCl_3$) : δ = 7,5 - 6,4 (m,11H); 4,2 (+,2H); 3,4 (s,3H); 3,2 (s,2H); 3,0 - 2,3 (m,11H); 2,2 (s,6H); 2,0 - 1,5 (m,4H); 1,1 + 0,9 (2d,6H) ppm.

Dihydrochlorid: Farblose Kristalle, Schmp. 258°C

| | | | | | |
|---|---|---|---|---|---|
| Ber. | $C_{36}H_{46}N_4O_3S \cdot 2HCl$ | C 62,9 | H 7,0 | N 8,1 | Cl 10,3 |
| Gef. | | C 63,1 | H 6,8 | N 8,0 | Cl 10,4 |

Chloressigsäure-2,6-dimethylanilid erhielt man aus Chloracetylchlorid und 2,6-Dimethylanilin unter Zusatz von Pyridin in Methylenchlorid. Farblose Kristalle, Schmp. 148 - 149°C (aus Petrolether).

Beispiel 10

3,4-Dihydro-2-isopropyl-4-methyl-2-[2-[4-[4-(N-methyl-carbamoyl)-piperazinyl]-butoxy]-phenyl]-2H-1,4-benzothiazin-3-on

4,5 g (10 mmol) der Verbindung aus Beispiel 3 wurden in 50 ml Methylenchlorid gelöst. 0,57 g Methylisocyanat wurden zugetropft. Nach 2 Std. bei Zimmertemperatur wurde eingeengt, und an Kieselgel chromatographiert. Man erhielt 4.0 g der Titelverbindung als farbloses Harz.

$^1$H-NMR ($CDCl_3$) : δ = 2,5 - 6,3 (m,8H); 5,1 (q,1H); 4,0 - 3,3 (m,6H); 3,4 (s,3H); 3,0 - 2,4 (m+d,10H); 2,0 - 1,6 (m,4H); 1,1 - 0,85 (2d,6H) ppm.

Beispiel 11

3,4-Dihydro-2-isopropyl-4-methyl-2-[2-[4-(4-butyryl)-piperazinyl)-butoxy]-phenyl]-2H-1,4-benzothiazin-3-on

4,5 g (10 mmol) der Verbindung aus Beispiel 3 wurden in 50 ml Methylenchlorid tropfenweise mit 1,06 g Butyrylchlorid versetzt. Nach 3 Stunden engt man ein und chromatographiert an Kieselgel. Man erhielt 3,3 g der Titelverbindung als farbloses Harz.

$^1$H-NMR ($CDCl_3$) : δ = 7,4 - 6,3 (m,8H); 4,0 - 3,6 (m,6H); 3,4 (s,3H); 3,0 - 2,6 (m,6H); 2,4 - 1,4 (m,9H); 1,2 + 0,8 (2d+t,9H) ppm.

Beispiel 12

3,4-Dihydro-2-isopropyl-4-methyl-2-[2-[4-[4-(3-methoxy-cinnamoyl]-piperazinyl]-butoxy]-phenyl]-2H-1,4-benzothiazin-3-on-Hydrochlorid

Eine Mischung aus 5 ml einer 30 %igen Lösung von 1-Hydroxybenztriazol, 3 g Dicyclohexylcarbodiimid, 4,6 gder Verbindung aus Beispiel 3 und 1,8 g 3-Methoxyzimtsäure wurde 4 Stunden bei Zimmertemperatur gerührt. Nach Absaugen wurde auf Wasser gegossen und mit Essigester extrahiert. Die organische Phase wurde 2 x mit Wasser gewaschen, mit Natriumsulfat getrocknet und eingeengt. Das Rohprodukt wurde an 300 g Kieselgel mit Methylenchlorid/Methanol (9:1) gereinigt. Man erhielt 4,7 g der freien Base als Öl.

$^1$H-NMR ($CDCl_3$) : $\delta$ = 8,0 - 6,5 (m,13H); 4,2 - 3,6 (m,6H); 3,8 (s,3H); 3,4 (s,3H); 3,3 - 2,8 (m,7H); 2,2 - 1,6 (m,4H); 1,15 - 0,95 (2d,6H) ppm.

Hydrochlorid: Farblose Kristalle vom Schmp. 80°C (Zers.)

Beispiel 13

3,4-Dihydro-2-isopropyl-4-methyl-2-[2-[4-(4-acetyl)-piperazinyl]-butoxy]-phenyl]-2H-1,4-benzothiazin-3-on-Hydrochlorid

4,5 g (10 mmol) der Verbindung aus Beispiel 3 wurden in 50 ml Methylenchlorid tropfenweise mit 1,02 g Acetanhydrid versetzt. Nach 3 Std. bei Zimmertemperatur engte man ein und chromatographierte an Kieselgel mit Methylenchlorid/Methanol (9:1) als Laufmittel. Man erhält 2,7 g der freien Base als Harz.

$^1$H-NMR ($CDCl_3$) : $\delta$ = 7,5 - 6,4 (m,8H); 4,0 - 3,4 (m,6H); 3,4 (s,3H); 3,0 - 2,2 (m,7H); 2,0 (s,3H); 2,0 - 1,6 (m,4H); 1,1 + 0,8 (2d,6H) ppm.

Hydrochlorid: Farblose Kristalle vom Schmp. 100°C (Zers.)

Beispiel 14

3,4-Dihydro-2-isopropyl-4-methyl-2-[2-[4-[4-(3-methoxy-anilino-carbonyl)-methyl]-piperazinyl]-butoxy]-phenyl]-2H-1,4-benzothiazin-3-on-Dihydrochlorid

45 g der Verbindung aus Beispiel 3 wurden mit 2 g Chloracetyl-3-methoxyanilid nach dem in Beispiel 9 angegebenen Verfahren umgesetzt. Man erhielt 4,9 g der freien Base als farbloses Öl.

$^1$H-NMR ($CDCl_3$) : δ = 7,5 - 6,6 (m,12H); 3,7 (s,3H); 3,4 (s,3H); 4,0 - 3,0 (m,13H); 2,1 - 1,6 (m,4H); 1,1 - 0,7 (2d,6H) ppm.

Dihydrochlorid: Farblose Kristalle, Schmp. 234 - 236°C (Zers.)

| | | | | | |
|---|---|---|---|---|---|
| Ber. | $C_{35}H_{44}N_4O_4S \cdot 2HCl \cdot H_2O$ | C 59,4 | H 6,8 | N 7,9 | Cl 10,0 |
| Gef. | | C 59,6 | H 6,6 | N 7,9 | Cl 10,2 |

Beispiel 15

3,4-Dihydro-2-isopropyl-4-methyl-2-[2-[4-[4-(2,6-dimethoxy-phenoxy)-acetyl]-piperazinyl]--butoxy]-phenyl]-2H-1,4-benzothiazin-3-on-Hydrochlorid

45 g (10 mmol) 3,4-Dihydro-2-isopropyl-4-methyl-2-[2-(4-brombutoxy)-phenyl] 2H-1,4-benzothiazin-3-on und 3,1 g 1-(2,6-Dimethoxyphenoxy)-acetyl-piperazin sowie 1,5 g gemahlenes, trockenes Kaliumcarbonat wurden in 30 ml DMF 6 Std. bei 90 - 100°C gerührt. Man verteilte das Reaktionsgemisch zwischen Wasser und Essigester, trennte die Phasen, wusch die Essigester-Phase mit Wasser und trocknete über Magnesiumsulfat. Nach Einengen wurde mit Essigester

am Kieselgel gereinigt. Man erhielt 4,3 g der freien Base als Harz.

$^{1}$H-NMR ($CDCl_3$) : δ = 7,4 - 6,3 (m,11H); 4,5 (s,2H); 4,0 - 3,6 (m,6H); 3,8 (s,6H); 3,4 (s,3H); 3,0 - 2,3 (m,7H); 2,0 - 1,6 (m,4H); 11 + 0,9 (2d,6H) ppm.

Hydrochlorid: Farblose Kristalle, Schmp. 145-148°C

| | | | | | |
|---|---|---|---|---|---|
| Ber. | $C_{36}H_{45}N_3O_6S \cdot HCl$ | C 63,2 | H 6,8 | N 6,1 | Cl 5,2 |
| Gef. | | C 62,8 | H 6,7 | N 6,1 | Cl 5,5 |

1-(2,6-Dimethoxyphenoxy)-acetylpiperazin erhielt man wie folgt:

125 g (80 mmol) 2,6-Dimethoxyphenoxyessigsäure wurden mit N-Benzylpiperazin (14,1 g) nach der Dicyclohexylcarbodiimid/Hydroxybenztriazol-Methode umgesetzt (Beispiel 5). Das so erhaltene 1-Benzyl-4-(2,6-dimethoxyphenoxy)-acetylpiperazin (18 g) wurden in 180 ml Methanol mit 1,8 g Palladium auf Kohle (10 %) als Katalysator bei 100 bar und 80°C 8 Stunden hydriert. Nach Filtration und Einengen erhielt man 6,6 g 1-(2,6-Dimethoxyphenoxy)-acetyl-piperazin, Schmp. 174 - 175°C (aus Aceton/Essigester).

Beispiel 16

3,4-Dihydro-2-isopropyl-4-methyl-2-[2-(oxiranyl-methoxy)-phenyl]-2H-1,4-benzothiazin-3-on

4,7 g (15 mmol) 3,4-Dihydro-2-isopropyl-4-methyl-2-(2-hydroxyphenyl)-2H-1,4-benzothiazin-3-on wurden mit 0,6 g NaOH, 2 ml $H_2O$ und 50 ml Epichlorhydrin 24 Std. bei 85°C gerührt. Nach Verdünnen mit Wasser wurde mit Methylenchlorid extrahiert, mit Natriumsulfat getrocknet und eingeengt. Man erhielt 5,3 g der Titelverbindung als Öl.

$^1$H-NMR ($CDCl_3$) : δ = 7,6 - 6,4 (m,8H); 4,2 - 3,8 (m,3H); 3,4 (s,3H); 3,4 - 2,5 (m,4H); 1,4 - 0,7 (m,6H) ppm.

Beispiel 17

3,4-Dihydro-2-isopropyl-4-methyl-2-[2-[2-hydroxy-3-[4-(3,4,5-trimethoxy-phenyl)-acetyl]-piperazinyl]-propoxy]-phenyl]-2H-1,4-benzothiazin-3-on-Hydrochlorid

5,5 g der Verbindung aus Beispiel 16 wurden mit 8 g 1-(3,4,5-Trimethoxyphenyl)-acetyl-piperazin in 50 ml Methanol am Rückfluß gekocht (10 Stunden). Nach Einengen wurde an 300 g Kieselgel mit Methylenchlorid/Methanol (9:1) chromatographiert. Man erhielt 4,7 g der freien Base als farblosen Harz.

$^1$H-NMR ($CDCl_3$) : δ = 7,5 - 6,4 (m,8H); 6,3 (s,2H); 3,8 (s,9H); 4,0 - 3,5 (m,9H); 3,4 (s,3H); 2,7 - 2,3 (m,5H); 1,3 - 0,8 (m,6H) ppm.

Hydrochlorid: Farbloses amorphes Pulver, F. 85°C (Zers.)

| | | | | |
|---|---|---|---|---|
| Ber. | $C_{36}H_{45}N_3O_7S \cdot 2HCl$ | C 58,7 | H 6,8 | N 5,7 |
| Gef. | | C 58,7 | H 6,9 | N 5,9 |

Beispiel 18

3,4-Dihydro-2-isopropyl-4-methyl-2-[2-[2-hydroxy-3-[4-[3-(3,4,5-trimethoxyphenyl)propionyl]-piperazinyl]-butoxy]-phenyl]-2H-1,4-benzothiazin-3-on

5,5 g der Verbindung aus Beispiel 16 wurden mit 8,4 g 1-[3-(3,4,5-Trimethoxyphenyl)-propionyl]-piperazin nach dem in Beispiel 17 angegebenen Verfahren umgesetzt. Man erhielt 6,8 g der Titelverbindung.

$^1$H-NMR ($CDCl_3$) : δ = 7,5 - 6,4 (m,8H); 6,3 (s,2H); 3,8 (s,9H); 4,0 - 3,5 (m,9H); 3,4 (s,3H); 2,7 - 2,3 (m,5H); 2,0 - 1,8 (m,2H); 1,3 - 0,6 (m,6H) ppm

Beispiel 19

3,4-Dihydro-2-isopropyl-4-methyl-2-[2-[4-[4-[3-(3,4,5-trimethoxyphenyl)-propionyl]-4-aminopiperidinyl]-butoxy]-phenyl]-2H-1,4-benzothiazin-3-on-Hydrochlorid

6,68 g (15 mmol) 3,4-Dihydro-2-isopropyl-4-methyl-2-[2-(4-brombutoxy)-phenyl]-2H-1,4-benzothiazin-3-on wurden mit 2,07 g gemahlenem, wasserfreiem Kaliumcarbonat und 4,83 g 4-[3-(3,4,5-Trimethoxyphenyl)-propionyl-amino]-piperidin 3 Std. in 30 ml DMF auf 80 - 90°C erhitzt. Man gab auf 300 ml Eiswasser, extrahierte mit Essigester (2 x), wusch die vereinigten organischen Phasen mit Wasser (2 x), trocknete über Natriumsulfat und engte ein. Man erhielt 9,8 g der freien Base, Schmp. 151 - 153°C.

$^1$H-NMR ($CDCl_3$) : δ = 7,4 - 6,4 (m,8H); 6,3 (s,2H); 5,4 (d,1H); 4,0 - 3,6 (m,3H); 3,7 (s,9H); 3,4 (s,3H); 3,0 - 2,2 (m,11H); 2,0 - 1,5 (m,4H); 1,1 + 0,9 (2d,6H) ppm.

Hydrochlorid: Farblose Kristalle, Schmp. 138-140°C (aus Ethanol)

| | | | | |
|---|---|---|---|---|
| Ber. | $C_{39}H_{51}N_3O_6S \cdot HCl \cdot H_2O$ | C 62,9 | H 7,3 | N 5,6 |
| Gef. | | C 63,4 | H 7,5 | N 5,8 |

4-[3-(3,4,5-Trimethoxyphenyl)-propionyl-amino]-piperidin erhielt man wie folgt:

24 g 3-(3,4,5-Trimethoxyphenyl)-propionsäure wurden mit 20,2 ml 4-Amino-1-benzylpiperidin nach dem Dicyclohexylcarbodiimid/1-Hydroxybenzotriazol-Verfahren (Beispiel 5)

umgesetzt. Das erhaltene 1-Benzyl-4-[3-(3,4,5-Trimethoxyphenyl)-propionyl-amino]-piperidin (36,1 g, Schmp. 93-95°C) wurde in 500 ml Methanol mit Pd/C (10 %, 5 g) bei 100 bar $H_2$-Druck und 80°C 12 Stunden hydriert. Nach Filtrieren und Einengen erhielt man 26 g vom Schmp. 110°C.

Beispiel 20

3,4-Dihydro-2-isopropyl-4-methyl-2-[2-[2-[4-[4-(3,4,5-trimethoxyphenyl)-acetyl-amino]-piperidinyl]-butoxy]-phenyl]-2H-1,4-benzothiazin-3-on-Hydrochlorid

4,5 g (10 mmol) 3,4-Dihydro-2-isopropyl-4-methyl-2-[2-(4-brombutoxy)-phenyl]-2H-1,4-benzothiazin-3-on wurden mit 3,1 g 4-(3,4,5-Trimethoxyphenyl-acetyl-amino)-piperidin nach dem in Beispiel 19 angegebenen Verfahren umgesetzt. Man erhielt 3,8 g freie Base als Öl.

$^1$H-NMR ($CDCl_3$) : δ = 7,5 - 6,6 (m,8H); 6,3 (s,2H); 5,65 (s,1H); 4,0 - 3,6 (m,3H); 3,9 (s,9H); 3,4 (s,3H); 3,5 - 1,5 (m,17H); 1,15 - 0,9 (2d,6H) ppm.

Hydrochlorid: Farbloses amorphes Pulver, Schmp. 85°C (Zers.)

4-(3,4,5-Trimethoxyphenylacetyl-amino)-piperidin erhielt man, wie in Beispiel 19 beschrieben, aus 3,4,5-Trimethoxyphenylessigsäure.

Beispiel 21

3,4-Dihydro-2-isopropyl-4-methyl-2-[2-[4-[4-(4-fluor-benzoyl)-piperidinyl]-butoxy]-phenyl]-2H-1,4-benzo-thiazin-3-on

4,5 g (10 mmol) 3,4-Dihydro-2-isopropyl-4-methyl-2-[2-(4-brombutoxy)-phenyl]-2H-1,4-benzothiazin-3-on wurden mit

2,45 g 4-(4-Fluorbenzoyl)-piperidin-Hydrochlorid und 1,2 g NaOH in 100 ml Methanol 2 Stunden bei 40°C gerührt, dann 3 Stunden zum Sieden erhitzt. Nach Einengen wurde zwischen Essigester und Wasser verteilt. Die organische Phase wurde mit Wasser gewaschen, mit Natriumsulfat getrocknet und eingeengt. Chromatographie am Kieselgel mit Methylenchlorid/ Methanol (9:1) als Laufmittel ergab 2,1 g hellbraunes Harz.

$^1$H-NMR ($CDCl_3$) : $\delta$ = 8,1 - 6,5 (m,12H); 3,9 (m,3H); 3,4 (s,3H); 3,3 - 2,1 (m,7H); 2,1 - 1,6 (m,8H); 1,1 + 0,95 (2d,6H) ppm.

Beispiel 22

3,4-Dihydro-2-isopropyl-4-methyl-2-[2-[2-hydroxy-3-[4-(3-methoxyanilinocarbonyl)-methyl]-piperazinyl]-propoxy]-phenyl]-2H-1,4-benzothiazin-3-on-Dihydrochlorid

6,6 g (18 mmol) der Verbindung aus Beispiel 16 wurden mit 9,1 g (37 mmol) 1-(3-Methoxy-anilino-carbonyl)-methylpiperazin und 70 ml Methanol 8 Std. zum Sieden erhitzt. Nach Einengen wurde an 300 g Kieselgel mit Methylenchlorid/ Methanol (9:1) chromatographiert. Man erhielt 2,7 g der freien Base als bräunliches, zähes Öl.

$^1$H-NMR ($CDCl_3$) : $\delta$ = 7,5 - 6,4 (m,12H); 4,2 - 3,3 (m,6H); 3,7 (m,3H); 3,4 (s,3H); 3,1 (s,2H); 2,8 - 2,4 (m,8H); 1,4 - 1,0 (m,5H); 0,7 (d,3H) ppm.

Dihydrochlorid: Farblose Kristalle, Schmp. 198-200°C (Zers.)

| | | | | |
|---|---|---|---|---|
| Ber. | $C_{34}H_{42}N_4O_5S \cdot 2HCl \cdot H_2O$ | C 57,5 | H 6,5 | N 7,9 |
| Gef. | | C 56,8 | H 6,0 | N 7,1 |

## Beispiel 23

3,4-Dihydro-2-benzyl-4-methyl-2-[2-[4-[-4-[3-(3,4,5-trimethoxyphenyl)-propionyl]-piperazinyl]-butoxy]-phenyl]-2H-1,4-benzothiazin-3-on-Hydrochlorid

5 g (10 mmol) 3,4-Dihydro-2-benzyl-4-methyl-2-[2-(4-brombutoxy)-phenyl]-2H-1,4-benzothiazin-3-on, 1,4 g Kaliumcarbonat und 6 g 1-[3-(3,4,5-Trimethoxyphenyl)-propionyl]-piperazin wurden in 100 ml DMF 16 Std. am Rückfluß gekocht. Nach Filtration wurde mit Essigester verdünnt, mit Wasser gewaschen (3 x), mit Natriumsulfat getrocknet und eingeengt. Chromatographie an Kieselgel ergab 3,3 g der freien Base.

$^1$H-NMR ($CDCl_3$) : $\delta$ = 7,3 - 6,5 (m,13H); 6,4 (s,2H); 3,8 (s,9H); 4,0 - 3,6 (m,8H); 3,4 (s,3H); 3,0 -2,2 (m,10H); 2,0 - 1,6 (m,4H) ppm.

Hydrochlorid: Farblose Kristalle, Schmp. 98-100°C (Zers.)

Die Herstellung von 3,4-Dihydro-2-benzyl-4-methyl-2-[2-(4-brombutoxy)-phenyl]-2H-1,4-benzothiazin-3-on ist in EP-A-146 893 beschrieben.

## Beispiel 24

3,4-Dihydro-2-benzyl-4-methyl-2-[2-[4-[-4-(3,4,5-trimethoxyphenyl)-acetylamino]-piperidinyl]-butoxy]-phenyl]-2H-1,4-benzothiazin-3-on-Hydrochlorid

5 g 3,4-Dihydro-2-benzyl-4-methyl-2-[2-(4-brombutoxy)-phenyl]-2H-1,4-benzothiazin-3-on wurden mit 3,1 g 4-(3,4,5-Trimethoxyphenyl)-acetylamino-piperidin analog der in Beispiel 23 angegebenen Vorschrift umgesetzt. Man erhielt 7,2 g der freien Base als Öl.

$^1$H-NMR ($CDCl_3$) : $\delta$ = 7,3 - 6,2 (m,15H); 4,1 - 3,3 (m,7H); 3,8 (s,9H); 3,4 (s,3H); 3,2 - 2,2 (m,6H); 2,1 - 1,5 (m,8H) ppm.

Hydrochlorid: Farblose Kristalle, Schmp. 115°C (Zers.)

Beispiel 25

3,4-Dihydro-2-benzyl-4-methyl-2-[2-[4-[-4-[3-(3-(3,4,5-trimethoxy-phenyl)-propionyl]-4-aminopiperidinyl] -butoxy] -phenyl]-2H-1,4-benzothiazin-3-on-Hydrochlorid

Hergestellt aus 5 g 3,4-Dihydro-2-benzyl-4-methyl-2-[2-(4-brombutoxy)-phenyl]-2H-1,4-benzothiazin-3-on und 3,1 g 4-[3-(3,4,5-Trimethoxyphenyl)-propionyl-amino]-piperidin nach der in Beispiel 23 angegebenen Vorschrift. Freie Base: farbloses Harz, Ausbeute 3,2 g

$^1$H-NMR ($CDCl_3$) : $\delta$ = 7,2 - 6,2 (m,15H); 4,1 - 3,3 (m,7H); 3,8 (s,9H); 3,4 (s,3H); 3,2 - 2,2 (m,6H); 2,1 - 1,5 (m,10H) ppm.

Hydrochlorid: Farblose Kristalle, Schmp. 114°C (Zers.)

0244723
HOE 86/F 088

Ansprüche:

1. Verbindung der Formel I sowie deren Salze mit pharmazeutisch akzeptablen Säuren,

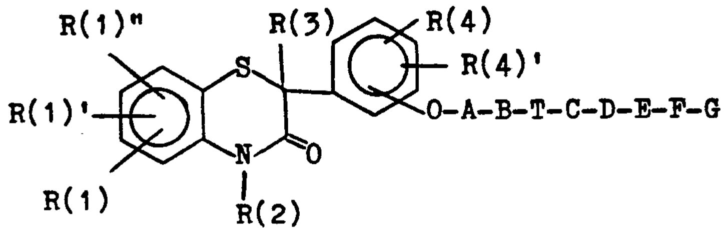

I

in welcher bedeuten:

R(1), R(1)' und R(1)" gleich oder verschieden und voneinander unabhängig Wasserstoff, $(C_1-C_4)$-Alkyl, $(C_1-C_3)$-Alkoxy, F, Cl, Br, $CF_3$, Nitro, Hydroxy, Acetamido oder Amino,

R(2) Wasserstoff, $(C_1-C_{10})$-Alkyl, geradkettig oder verzweigt, $(C_3-C_{10})$-Alkenyl, geradkettig oder verzweigt, Phenyl-$(C_1-C_4)$-alkyl, wobei der Phenylring unsubstituiert oder durch einen, zwei oder drei Substituenten aus der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(3) Wasserstoff, $(C_1-C_{15})$-Alkyl, geradkettig oder verzweigt, $(C_3-C_{15})$-Alkenyl, geradkettig oder verzweigt, $(C_4-C_8)$-Cycloalkyl, $(C_4-C_8)$-Cycloalkyl-$(C_1-C_4)$alkyl, Phenyl oder Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)', gleich oder verschieden und voneinander unabhängig, Wasserstoff, $(C_1-C_4)$-Alkyl, $(C_1-C_3)$-Alkoxy, F, Cl, $CF_3$, Nitro, Hydroxy, Acetamido oder Amino,

A die Gruppe $(CH_2)_m-X-(CH_2)_n$, wobei

m 1, 2, 3 oder 4, wobei jedoch m, wenn X ein Heteroatom ist, nur 2, 3 oder 4 ist,

n Null, 1, 2 oder 3, sofern X ein Heteroatom ist, jedoch nur 2 oder 3,

X eine $CH_2$-Gruppe, Sauerstoff, Schwefel, eine Carbonylgruppe, eine CH(OH)-Gruppe oder eine Gruppe

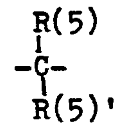 , worin R(5) und

R(5)' gleich oder verschieden sind und Wasserstoff oder $(C_1-C_4)$-Alkyl bedeuten,

B eine der folgenden Gruppen

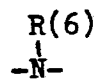 , 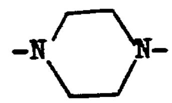 , 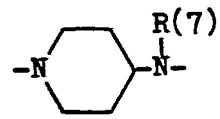

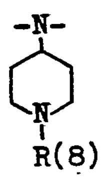 , 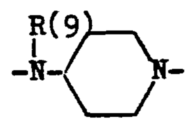 , 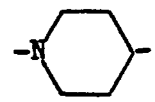 ,

T einen $(CH_2)_p$-Rest, wobei p Null, 1 oder 2 ist, wobei jedoch p, wenn D die CHOH-Gruppe bedeutet, nur 1, 2 oder 3 ist, und wobei p, wenn D ein Heteroatom bedeutet, nur 2 oder 3 bedeutet,

D eine CH(OH)-Gruppe, eine Carbonylgruppe, eine -NR(10)-CO-Gruppe, eine CO-NR(11)-Gruppe oder ein Sauerstoffatom

E einen $(CH_2)_q$-Rest, wobei q Null, 1, 2 oder 3 ist, oder einen -CH=CH-Rest, jedoch, wenn D und/oder F ein Heteroatom bedeutet, E nur $(CH_2)_q$ mit q= 2 oder 3 bedeutet,

F eine Einfachbindung, einen NR(12)-CO-Rest, einen CO-NR(13)-Rest, eine Carbonylgruppe oder ein Sauerstoffatom

G Phenyl, wobei der Phenylring unsubstituiert oder durch einen, zwei oder drei Substituenten der Gruppe $(C_1-C_4)$-Alkyl, $(C_1-C_3)$-Alkoxy, F, Cl, $(C_1-C_2)$-Alkylendioxy oder Cyano substituiert ist, oder 2-Furyl

R(6) und R(7) und R(8) und R(9) Wasserstoff, $(C_1-C_{10})$-Alkyl, geradkettig oder verzweigt, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl, oder Benzyl, wobei die Phenylreste jeweils unsubstituiert oder

durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(10) und R(11) und R(12) und R(13) Wasserstoff oder $(C_1-C_5)$-Alkyl;

2. Verbindung I nach Anspruch 1, dadurch gekennzeichnet, daß mindestens einer der Substituenten und Indices folgende Bedeutung hat:

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido,

R(1)" Wasserstoff,

R(2) Wasserstoff, $(C_1-C_6)$-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, $(C_1-C_{12})$-Alkyl, geradkettig oder verzweigt, Allyl, Methallyl, $(C_5-C_7)$Cycloalkyl, $(C_5-C_7)$-Cycloalkyl-$(C_1-C_4)$-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)' Wasserstoff,

A die Gruppe $(CH_2)_m-X-(CH_2)_n$, wobei

m 1, 2, 3 oder 4 wobei jedoch m, wenn X ein Heteroatom ist, nur 2, 3 oder 4 ist,

n Null, 1, 2 oder 3, sofern X ein Heteroatom bedeutet, jedoch nur 2 oder 3,

X eine $CH_2$-Gruppe, Sauerstoff, Schwefel, eine Carbonylgruppe eine CH(OH)-Gruppe oder eine Gruppe R(5)-C-R(5)', worin R(5) und

R(5)' gleich oder verschieden sind und Wasserstoff, Methyl oder Ethyl bedeuten,

B eine der folgenden Gruppen

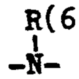 , 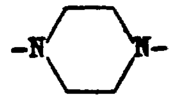 , 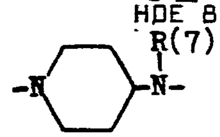

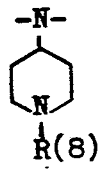 , 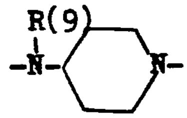 , 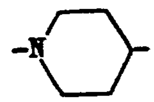 ,

T einen $(CH_2)_p$-Rest, wobei p Null, 1 oder 2 ist, wobei jedoch p, wenn D die CHOH-Gruppe bedeutet, nur 1, 2 oder 3 ist, und wobei p, wenn D ein Heteroatom bedeutet, nur 2 oder 3 bedeutet,

D eine CH(OH)-Gruppe, eine Carbonylgruppe, eine -NR(10)-CO-Gruppe, eine CO-NR(11)-Gruppe oder ein Sauerstoffatom,

E einen $(CH_2)_q$-Rest, wobei q Null, 1 oder 2 ist, oder einen -CH=CH-Rest,

F eine Einfachbindung, einen NR(12)-CO-Rest, einen CO-NR(13)-Rest, eine Carbonylgruppe oder ein Sauerstoffatom,

G Phenyl oder 2-Furyl, wobei der Phenylring unsubstituiert oder durch ein oder zwei Substituenten aus der Gruppe $(C_1-C_2)$-Alkyl, F, Cl, $(C_1-C_2)$-Alkylendioxy oder Cyano oder durch ein, zwei oder drei $(C_1-C_2)$-Alkoxy-Substituenten substituiert ist,

R(6), R(7), R(8), R(9), R(10), R(11) und R(12) und R(13) Wasserstoff oder $(C_1-C_4)$-Alkyl.

3. Verbindung I nach Anspruch 1, dadurch gekennzeichnet, daß mindestens einer der Substituenten und Indices folgende Bedeutung hat:

R(1) Wasserstoff, Methyl, Methoxy, Fluor oder Chlor,
R(1)' Wasserstoff oder Methoxy,
R(1)" Wasserstoff,
R(2) Wasserstoff, Methyl, Ethyl, Propyl, Isopropyl, Butyl, sec. Butyl, Isobutyl, Benzyl, Phenethyl,
R(3) Wasserstoff $(C_1-C_{12})$-Alkyl, geradkettig oder verzweigt, Cyclopentyl, Cyclohexyl, Cyclopentylmethyl,

Cyclohexylmethyl, Allyl, Methallyl, Benzyl, Methylbenzyl, Fluorbenzyl, Methoxybenzyl, Dimethoxybenzyl, Phenylethyl,

R(4) Wasserstoff, Methoxy, Methyl, Chlor, Nitro oder Hydroxy,

R(4)' Wasserstoff

A die Gruppe $(CH_2)_m-X-(CH_2)_n$, worin

m 1, 2 oder 3 wobei jedoch m, wenn X ein Heteroatom ist, nur 2, 3 oder 4 ist,

n 0, 1 oder 2, sofern X ein Heteroatom bedeutet, jedoch nur 2

X eine $CH_2$-Gruppe, Sauerstoff, eine Carbonylgruppe eine CH(OH)-Gruppe oder eine Gruppe

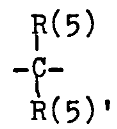 worin R(5) und

R(5)' gleich oder verschieden sind und Wasserstoff oder Methyl bedeuten,

B eine der folgenden Gruppen

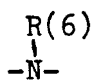 , 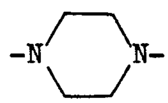 , 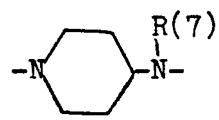

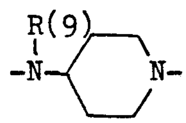 , 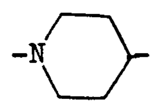

T einen $(CH_2)_p$-Rest, wobei p Null, 1 oder 2 ist, wobei jedoch p, wenn D die CHOH-Gruppe bedeutet, nur 1, 2 oder 3 ist, und wobei p, wenn D ein Heteroatom bedeutet, nur 2 oder 3 bedeutet,

D eine CH(OH)-Gruppe, eine Carbonylgruppe, eine -NR(10)-CO-Gruppe, eine CO-NR(11)-Gruppe oder ein Sauerstoffatom,

E ein $(CH_2)_q$-Rest, wobei q Null, 1 oder 2 ist,

F eine Einfachbindung, einen NR(12)-CO-Rest, einen CO-NR(13)-Rest, eine Carbonylgruppe oder ein Sauerstoffatom,

G Phenyl, wobei der Phenylring unsubstituiert oder durch 1 oder 2 Reste der Gruppe Methyl, F, Cl,

Methylendioxy oder Cyano oder durch 1, 2 oder 3 Methoxyreste substituiert ist, oder 2-Furyl

R(6), R(7), R(8), R(9), R(10), R(11), R(12) und R(13) gleich oder verschieden sind und Wasserstoff oder $(C_1-C_3)$-Alkyl bedeuten.

4. Verfahren zum Herstellen einer Verbindung I nach Anspruch 1, dadurch gekennzeichnet, daß man

a) eine Verbindung der Formel II,

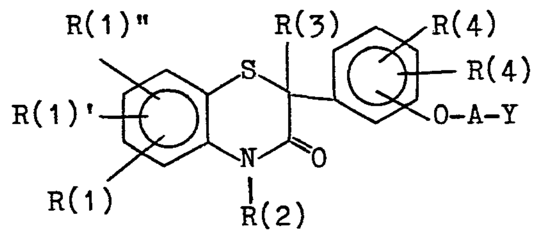

II,

in welcher R(1), R(1)', R(1)", R(2), R(3), R(4), R(4)' und A die gleiche Bedeutung wie in Formel I haben, und in welcher Y eine Abgangsgruppe, die nucleophil verdrängt werden kann, bedeutet, mit einer Verbindung der Formel III

H-B-T-D-E-F-G    III,

in welcher B, T, D, E, F und G die gleiche Bedeutung wie in Formel I haben, unter Bedingungen einer nucleophilen Substitution, mit oder ohne Gegenwart einer Hilfsbase zum Abfangen der sich bildenden Säure, bei einer Temperatur zwischen 0 und 160°C, umsetzt, oder daß man

b) eine Verbindung der Formel IV,

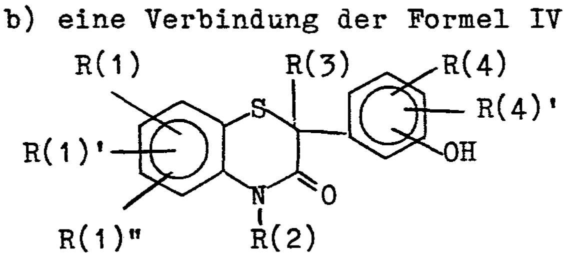

IV,

in welcher R(1), R(1)', R(1)", R(2), R(3), R(4) und R(4)' die gleiche Bedeutung wie in Formel I haben, mit einer Verbindung der Formel V,

Z-A-B-T-D-E-F-G    V,

in welcher Z gleich wie Y in Formel II definiert ist und in welcher A, B, T, D, E, F und G die gleiche Bedeutung wie in Formel I haben, entweder in einem polaren aprotischen Lösungsmittel in Gegenwart einer starken Base bei einer Temperatur zwischen -40 und +60°C, oder in einem protischen oder aprotischen polaren organischen Lösungsmittel in Gegenwart einer schwachen bis mittelstarken Base bei einer Temperatur zwischen 0 und 160°C, umsetzt, oder daß man

c) eine Verbindung der Formel V,

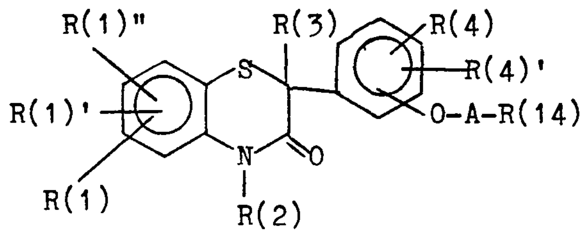

V

in welcher R(1), R(1)', R(1)", R(2), R(3), R(4), R(4)', und A die gleiche Bedeutung wie in Formel I haben und in welcher R(14) eine der folgenden Gruppen

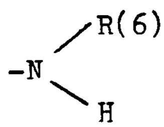 , 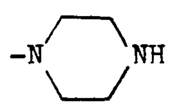 , 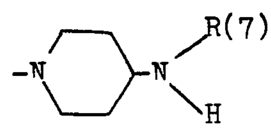 ,

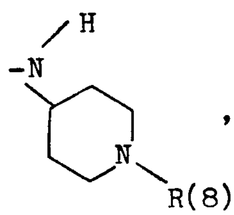 , 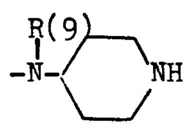

bedeutet, worin R(6), R(7), R(8) und R(9) die gleiche Bedeutung wie in Formel I haben, mit einer Verbindung der Formel VI

$$W-\underset{\underset{O}{\|}}{C}-E-F-G \qquad VI,$$

in welcher E, F und G die gleiche Bedeutung wie in Formel I haben, und W ein Chloratom, eine $(C_1-C_5)$-Alkoxygruppe, eine $(C_1-C_5)$-Alkanoyloxygruppe oder ein Imidazolrest ist, unter üblichen Amidbildungsbedingungen umsetzt, wobei Verbindungen der Formel I gebildet werden, in denen p Null und D eine Carbonylgruppe bedeuten, oder daß man

d) eine Verbindung der Formel VII

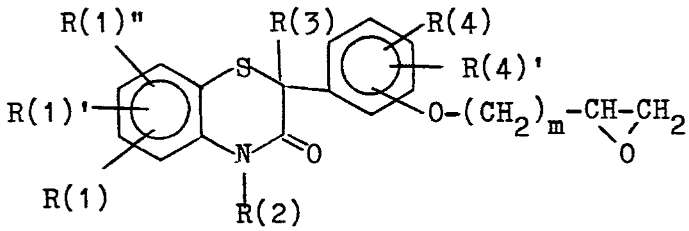 VII,

in welcher R(1), R(1)', R(1)", R(2), R(3), R(4), R(4)' und m die gleiche Bedeutung wie in Formel I haben, mit einer Verbindung der Formel VIII

H-B-T-D-E-F-G VIII,

in welcher B, T, D, E, F, G wie in Formel I definiert sind, ohne Lösungsmittel oder in Gegenwart eines vorzugsweise polaren Lösungsmittels umsetzt, wobei Verbindungen der Formel I entstehen, worin A den Rest $-(CH_2)_m-CH(OH)-CH_2-$ bedeutet, oder daß man

e) eine Verbindung der Formel V mit einer Verbindung der Formel IX

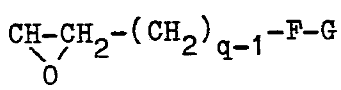 IX

worin F, G und q die gleiche Bedeutung wie in Formel I haben, unter den unter d beschriebenen Reaktionsbedingungen umsetzt, wobei Verbindungen der Formel I gebildet werden, in denen p gleich 1 und D eine CH(OH)-Gruppe bedeuten.

5. Arzneimittel, dadurch gekennzeichnet, daß es eine Verbindung I nach Anspruch 1 enthält oder daraus besteht.

6. Verwendung einer Verbindung I nach Anspruch 1 zur Behandlung von Störungen im Calciumhaushalt eines menschlichen oder tierischen Organismus.

Patentansprüche Griechenland, Österreich und Spanien:

1. Verfahren zum Herstellen einer Verbindung I sowie von deren Salzen mit pharmazeutisch akzeptablen Säuren,

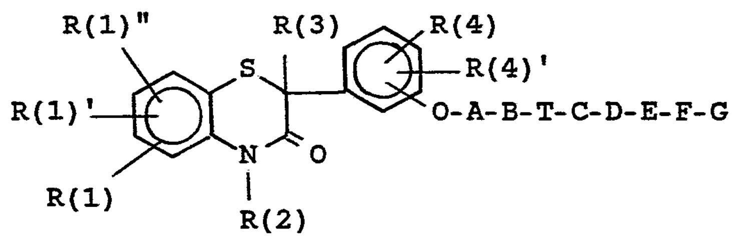

I

in welcher bedeuten:

R(1), R(1)' und R(1)" gleich oder verschieden und voneinander unabhängig Wasserstoff, $(C_1-C_4)$-Alkyl, $(C_1-C_3)$-Alkoxy, F, Cl, Br, $CF_3$, Nitro, Hydroxy, Acetamido oder Amino,

R(2) Wasserstoff, $(C_1-C_{10})$-Alkyl, geradkettig oder verzweigt, $(C_3-C_{10})$-Alkenyl, geradkettig oder verzweigt, Phenyl-$(C_1-C_4)$-alkyl, wobei der Phenylring unsubstituiert oder durch einen, zwei oder drei Substituenten aus der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(3) Wasserstoff, $(C_1-C_{15})$-Alkyl, geradkettig oder verzweigt, $(C_3-C_{15})$-Alkenyl, geradkettig oder verzweigt, $(C_4-C_8)$-Cycloalkyl, $(C_4-C_8)$-Cycloalkyl-$(C_1-C_4)$alkyl, Phenyl oder Phenyl-$(C_1-C_4)$-alkyl, wobei der Phenylrest unsubstituiert oder durch einen, zwei oder drei Substituenten der Gruppe $(C_1-C_4)$-Alkyl, $(C_1-C_3)$-Alkoxy, F, Cl, $CF_3$, $(C_1-C_2)$-Alkylendioxy oder Nitro substituiert ist,

R(4) und R(4)', gleich oder verschieden und voneinander unabhängig, Wasserstoff, $(C_1-C_4)$-Alkyl, $(C_1-C_3)$-Alkoxy, F, Cl, $CF_3$, Nitro, Hydroxy, Acetamido oder Amino,

A die Gruppe $(CH_2)_m-X-(CH_2)_n$, wobei

m 1, 2, 3 oder 4,
wobei jedoch m wenn X ein Heteroatom ist, nur 2, 3 oder 4 ist,

n Null, 1, 2 oder 3, sofern X ein Heteroatom ist, jedoch nur 2 oder 3,

X eine $CH_2$-Gruppe, Sauerstoff, Schwefel, eine Carbonylgruppe, eine CH(OH)-Gruppe oder eine Gruppe

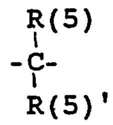 , worin R(5) und

R(5)' gleich oder verschieden sind und Wasserstoff oder $(C_1-C_4)$-Alkyl bedeuten,

B eine der folgenden Gruppen

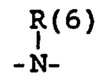 , 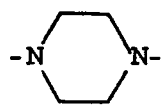 , 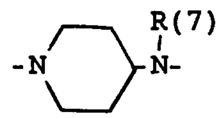

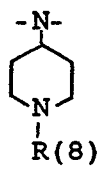 , 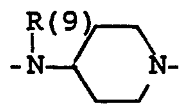 , 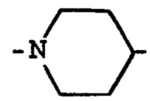

T einen $(CH_2)_p$-Rest, wobei p Null, 1 oder 2 ist, wobei jedoch p, wenn D die CHOH-Gruppe bedeutet, nur 1, 2 oder 3 ist, und wobei p, wenn D ein Heteroatom bedeutet, nur 2 oder 3 bedeutet,

D eine CH(OH)-Gruppe, eine Carbonylgruppe, eine -NR(10)-CO-Gruppe, eine CO-NR(11)-Gruppe oder ein Sauerstoffatom

E einen $(CH_2)_q$-Rest, wobei q Null, 1, 2 oder 3 ist, oder einen -CH=CH-Rest, jedoch, wenn D und/oder F ein Heteroatom bedeutet, E nur $(CH_2)_q$ mit q= 2 oder 3 bedeutet,

F eine Einfachbindung, einen NR(12)-CO-Rest, einen CO-NR(13)-Rest, eine Carbonylgruppe oder ein Sauerstoffatom

G Phenyl, wobei der Phenylring unsubstituiert oder durch einen, zwei oder drei Substituenten der Gruppe $(C_1-C_4)$-Alkyl, $(C_1-C_3)$-Alkoxy, F, Cl, $(C_1-C_2)$-Alkylendioxy oder Cyano substituiert ist, oder 2-Furyl

R(6) und R(7) und R(8) und R(9) Wasserstoff, $(C_1-C_{10})$-Alkyl, geradkettig oder verzweigt, $(C_1-C_6)$-Alkanoyl, Phenyl-$(C_1-C_4)$-alkyl, Benzhydryl oder Benzhydryl-$(C_1-C_4)$-alkyl, Phenyl-$(C_1-C_4)$-alkanoyl, oder Benzyl, wobei die Phenylreste jeweils unsubstituiert oder

durch einen, zwei oder drei Reste aus der Gruppe $(C_1-C_4)$-Alkyl, $(C_1-C_4)$-Alkoxy, $(C_1-C_2)$-Alkylendioxy, F, Cl, Br, $CF_3$ oder Hydroxy substituiert sind,

R(10) und R(11) und R(12) und R(13) Wasserstoff oder $(C_1-C_5)$-Alkyl, dadurch gekennzeichnet, daß man

a) eine Verbindung der Formel II,

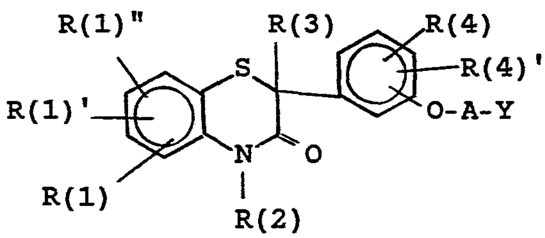

II

in welcher R(1), R(1)', R(1)", R(2), R(3), R(4), R(4)' und A die gleiche Bedeutung wie in Formel I haben, und in welcher Y eine Abgangsgruppe, die nucleophil verdrängt werden kann, bedeutet, mit einer Verbindung der Formel III

H-B-T-D-E-F-G III

in welcher B, T, D, E, F und G die gleiche Bedeutung wie in Formel I haben, unter Bedingungen einer nucleophilen Substitution, mit oder ohne Gegenwart einer Hilfsbase zum Abfangen der sich bildenden Säure, bei einer Temperatur zwischen 0 und 160°C, umsetzt, oder daß man

b) eine Verbindung der Formel IV,

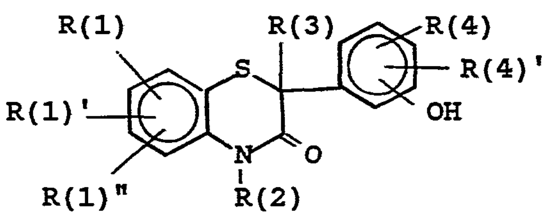

IV,

in welcher R(1), R(1)', R(1)", R(2), R(3), R(4) und R(4)' die gleiche Bedeutung wie in Formel I haben, mit einer Verbindung der Formel V,

Z-A-B-T-D-E-F-G V,

in welcher Z gleich wie Y in Formel II definiert ist und in welcher A, B, T, D, E, F und G die gleiche Bedeutung wie in Formel I haben, entweder in einem polaren aprotischen Lösungsmittel in Gegenwart einer starken Base bei einer Temperatur zwischen -40 und +60°C, oder in einem protischen oder aprotischen polaren organischen Lösungsmittel in Gegenwart einer schwachen bis mittelstarken Base bei einer Temperatur zwischen 0 und 160°C, umsetzt, oder daß man

c) eine Verbindung der Formel V,

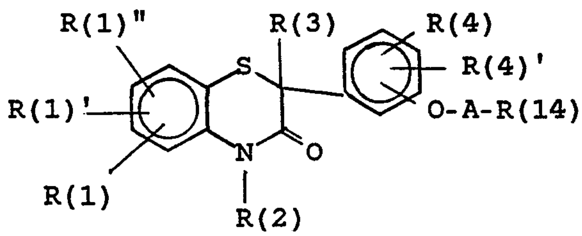 V

in welcher R(1), R(1)', R(1)", R(2), R(3), R(4), R(4)', und A die gleiche Bedeutung wie in Formel I haben und in welcher R(14) eine der folgenden Gruppen

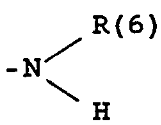 , 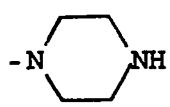 , 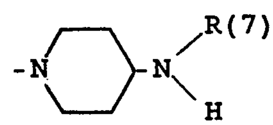 ,

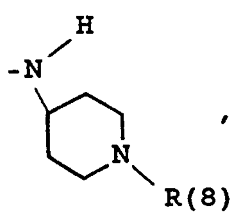 , 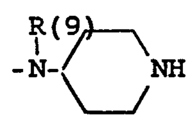

bedeutet, worin R(6), R(7), R(8) und R(9) die gleiche Bedeutung wie in Formel I haben, mit einer Verbindung der Formel VI

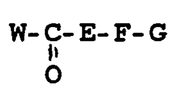 VI,

in welcher E, F und G die gleiche Bedeutung wie in Formel I haben, und W ein Chloratom, eine $(C_1-C_5)$-Alkoxygruppe, eine $(C_1-C_5)$-Alkanoyloxygruppe oder ein Imidazolrest ist, unter üblichen Amidbildungsbedingungen umsetzt, wobei Verbindungen der Formel I gebildet werden, in denen p Null und D eine Carbonylgruppe bedeuten, oder daß man

d) eine Verbindung der Formel VII

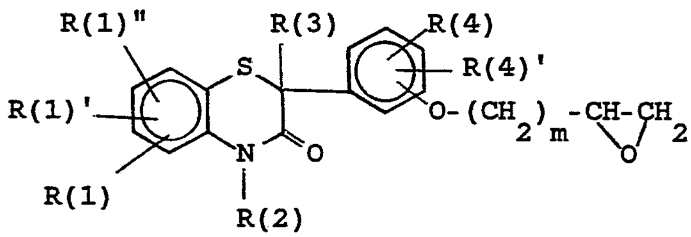

VII,

in welcher R(1), R(1)', R(1)", R(2), R(3), R(4), R(4)' und m die gleiche Bedeutung wie in Formel I haben, mit einer Verbindung der Formel VIII

H-B-T-D-E-F-G VIII,

in welcher B, T, D, E, F, G wie in Formel I definiert sind, ohne Lösungsmittel oder in Gegenwart eines vorzugsweise polaren Lösungsmittels umsetzt, wobei Verbindungen der Formel I entstehen, worin A den Rest $-(CH_2)_m-\underset{OH}{CH}-CH_2-$ bedeutet, oder daß man

e) eine Verbindung der Formel V mit einer Verbindung der Formel IX

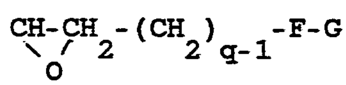

IX

worin F, G und q die gleiche Bedeutung wie in Formel I haben, unter den unter d beschriebenen Reaktionsbedingungen umsetzt, wobei Verbindungen der Formel I gebildet werden, in denen p gleich 1 und D eine CH(OH)-Gruppe bedeuten.

2. Verfahren nach Anspruch 1, dadurch gekennzeichnet, daß mindestens einer der Substituenten und Indices folgende Bedeutung hat:

R(1) und R(1)' gleich oder verschieden sind und voneinander unabhängig Wasserstoff, Methyl, Ethyl, Methoxy, Ethoxy, Fluor, Chlor, $CF_3$, Nitro oder Acetamido,

R(1)" Wasserstoff,

R(2) Wasserstoff, $(C_1-C_6)$-Alkyl, geradkettig oder verzweigt, Allyl, Methallyl, Benzyl, Phenethyl, 4-Methoxybenzyl, 3,4-Dimethoxybenzyl, 3,4,5-Trimethoxybenzyl, 3,4-Methylendioxybenzyl,

R(3) Wasserstoff, $(C_1-C_{12})$-Alkyl, geradkettig oder verzweigt, Allyl, Methallyl, $(C_5-C_7)$Cycloalkyl, $(C_5-C_7)$-Cycloalkyl-$(C_1-C_4)$-alkyl, Benzyl, Methylbenzyl, Fluorbenzyl, Methoxybenzyl, Dimethoxybenzyl, Phenylethyl,

R(4) Wasserstoff, Methyl, Methoxy, Ethoxy, Chlor, Nitro, Hydroxy, Acetamido oder Amino,

R(4)' Wasserstoff,

A die Gruppe $(CH_2)_m-X-(CH_2)_n$, wobei

m 1, 2, 3 oder 4 wobei jedoch m, wenn X ein Heteroatom ist, nur 2, 3 oder 4 ist,

n Null, 1, 2 oder 3, sofern X ein Heteroatom bedeutet, jedoch nur 2 oder 3,

X eine $CH_2$-Gruppe, Sauerstoff, Schwefel, eine Carbonylgruppe eine CH(OH)-Gruppe oder eine Gruppe R(5)-C-R(5)', worin R(5) und

R(5)' gleich oder verschieden sind und Wasserstoff, Methyl oder Ethyl bedeuten,

B eine der folgenden Gruppen

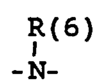 , 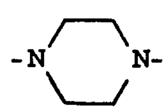 , 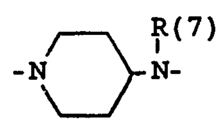

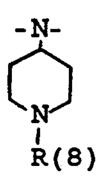 , 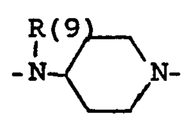 , 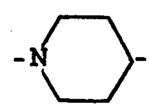

T einen $(CH_2)_p$-Rest, wobei p Null, 1 oder 2 ist, wobei jedoch p, wenn D die CHOH-Gruppe bedeutet, nur 1, 2 oder 3 ist, und wobei p, wenn D ein Heteroatom bedeutet, nur 2 oder 3 bedeutet,

D eine CH(OH)-Gruppe, eine Carbonylgruppe, eine -NR(10)-CO-Gruppe, eine CO-NR(11)-Gruppe oder ein Sauerstoffatom,

E einen $(CH_2)_q$-Rest, wobei q Null, 1 oder 2 ist, oder einen -CH=CH-Rest, jedoch, wenn D und/oder F ein Heteroatom bedeutet, E nur $(CH_2)_q$ mit q= 2 oder 3 bedeutet,

F eine Einfachbindung, einen NR(12)-CO-Rest, einen CO-NR(13)-Rest, eine Carbonylgruppe oder ein Sauerstoffatom,

G Phenyl oder 2-Furyl, wobei der Phenylring unsubstituiert oder durch ein oder zwei Substituenten aus der Gruppe $(C_1-C_2)$-Alkyl, F, Cl, $(C_1-C_2)$-Alkylendioxy oder Cyano oder durch ein, zwei oder drei $(C_1-C_2)$-Alkoxy-Substituenten substituiert ist,

R(6), R(7), R(8), R(9), R(10), R(11) und R(12) und R(13) Wasserstoff oder $(C_1-C_4)$-Alkyl.

3. Verfahren nach Anspruch 1, dadurch gekennzeichnet, daß mindestens einer der Substituenten und Indices folgende Bedeutung hat:

R(1) Wasserstoff, Methyl, Methoxy, Fluor oder Chlor,

R(1)' Wasserstoff oder Methoxy,

R(1)" Wasserstoff,

R(2) Wasserstoff, Methyl, Ethyl, Propyl, Isopropyl, Butyl, sec. Butyl, Isobutyl, Benzyl, Phenethyl,

R(3) Wasserstoff $(C_1-C_{12})$-Alkyl, geradkettig oder verzweigt, Cyclopentyl, Cyclohexyl, Cyclopentylmethyl, Cyclohexylmethyl, Allyl, Methallyl, Benzyl, Methylbenzyl, Fluorbenzyl, Methoxybenzyl, Dimethoxybenzyl, Phenylethyl,

R(4) Wasserstoff, Methoxy, Methyl, Chlor, Nitro oder Hydroxy,

R(4)' Wasserstoff

A die Gruppe $(CH_2)_m-X-(CH_2)_n$, worin

m 1, 2 oder 3 wobei jedoch m, wenn X ein Heteroatom ist, nur 2, 3 oder 4 ist,

n 0, 1 oder 2, sofern X ein Heteroatom bedeutet, jedoch nur 2

X eine $CH_2$-Gruppe, Sauerstoff, eine Carbonylgruppe eine CH(OH)-Gruppe oder eine Gruppe

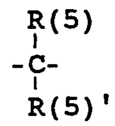 worin R(5) und

R(5)' gleich oder verschieden sind und Wasserstoff oder Methyl bedeuten,

B eine der folgenden Gruppen

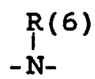 , 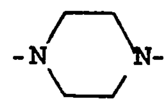 , 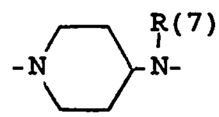

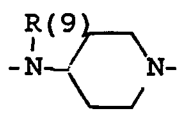 , 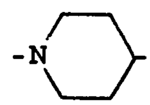

T einen $(CH_2)_p$-Rest, wobei p Null, 1 oder 2 ist, wobei jedoch p, wenn D die CHOH-Gruppe bedeutet, nur 1, 2 oder 3 ist, und wobei p, wenn D ein Heteroatom bedeutet, nur 2 oder 3 bedeutet,

D eine CH(OH)-Gruppe, eine Carbonylgruppe, eine -NR(10)-CO-Gruppe, eine CO-NR(11)-Gruppe oder ein Sauerstoffatom,

E ein $(CH_2)_q$-Rest, wobei q Null, 1 oder 2 ist,

F eine Einfachbindung, einen NR(12)-CO-Rest, einen CO-NR(13)-Rest, eine Carbonylgruppe oder ein Sauerstoffatom,

G Phenyl, wobei der Phenylring unsubstituiert oder durch 1 oder 2 Reste der Gruppe Methyl, F, Cl, Methylendioxy oder Cyano oder durch 1, 2 oder 3 Methoxyreste substituiert ist, oder 2-Furyl

R(6), R(7), R(8), R(9), R(10), R(11), R(12) und R(13)

gleich oder verschieden sind und Wasserstoff oder $(C_1-C_3)$-Alkyl bedeuten.

4. Verfahren zum Herstellen eines Arzneimittels, dadurch gekennzeichnet, daß man eine nach Anspruch 1 erhaltene Verbindung I mit pharmazeutisch üblichen Trägerstoffen mischt.

5. Verwendung einer nach Anspruch 1 erhaltenen Verbindung I zum Herstellen eines Medikaments zur Behandlung von Störungen im Calciumhaushalt eines menschlichen oder tierischen Organismus.